# EUROPEAN PATENT SPECIFICATION

(11) **EP 2 378 897 B1**
(45) Date of publication and mention of the grant of the patent: **19.07.2017**
(21) Application number: 09775207.5
(22) Date of filing: 16.12.2009
(51) Int. Cl.: A23L 5/20, A23L 19/12, A23L 19/18

(54) **STABILIZATION OF ASPARAGINASE**
STABILISIERUNG VON ASPARAGINASE
STABILISATION D'ASPARAGINASE

(30) Priority: 16.12.2008 EP 08171852
(43) Date of publication of application: 26.10.2011
(73) Proprietor: Novozymes A/S, 2880 Bagsvaerd (DK)
(72) Inventor: HENRIKSEN, Hanne, Vang, DK-2840 Holte (DK); ERNST, Steffen, DK-2700 Broenshoej (DK); BARFOED, Martin, DK-3060 Espergærde (DK)
(86) International application number: PCT/EP2009/067328
(87) International publication number: WO 2010/070010

(56) References cited:
- WO-A1-2004/026042
- WO-A1-2004/032648
- WO-A1-2007/073945
- WO-A1-2008/110513
- WO-A1-2008/128974
- WO-A2-2004/030468
- WO-A2-2006/053563
- DE-A1-102007 027 825

### SEQUENCE LISTING

<110> Novozymes A/S
<120> Stabilization of asparaginase
<130> 11533.204-WO
<160> 1
<170> PatentIn version 3.5
<210> 1
   <211> 378
   <212> PRT
   <213> Aspergillus oryzae
<400> 1
   Met Gly Val Asn Phe Lys Val Leu Ala Leu Ser Ala Leu Ala Thr Ile
   1               5                   10                  15
   Ser His Ala Ser Pro Leu Leu Tyr Pro Arg Ala Thr Asp Ser Asn Val
               20                  25                  30
   Thr Tyr Val Phe Thr Asn Pro Asn Gly Leu Asn Phe Thr Gln Met Asn
           35                  40                  45
   Thr Thr Leu Pro Asn Val Thr Ile Phe Ala Thr Gly Gly Thr Ile Ala
       50                  55                  60
   Gly Ser Ser Ala Asp Asn Thr Ala Thr Thr Gly Tyr Lys Ala Gly Ala
   65                  70                  75                  80
   Val Gly Ile Gln Thr Leu Ile Asp Ala Val Pro Glu Met Leu Asn Val
                   85                  90                  95
   Ala Asn Val Ala Gly Val Gln Val Thr Asn Val Gly Ser Pro Asp Ile
               100                 105                 110
   Thr Ser Asp Ile Leu Leu Arg Leu Ser Lys Gln Ile Asn Glu Val Val
           115                 120                 125
   Cys Asn Asp Pro Thr Met Ala Gly Ala Val Val Thr His Gly Thr Asp
       130                 135                 140
   Thr Leu Glu Glu Ser Ala Phe Phe Leu Asp Ala Thr Val Asn Cys Arg
   145                 150                 155                 160
   Lys Pro Val Val Ile Val Gly Ala Met Arg Pro Ser Thr Ala Ile Ser
                   165                 170                 175
   Ala Asp Gly Pro Leu Asn Leu Leu Gln Ser Val Thr Val Ala Ala Ser
               180                 185                 190
   Pro Lys Ala Arg Asp Arg Gly Ala Leu Ile Val Met Asn Asp Arg Ile
           195                 200                 205
   Val Ser Ala Phe Tyr Ala Ser Lys Thr Asn Ala Asn Thr Val Asp Thr
       210                 215                 220
   Phe Lys Ala Ile Glu Met Gly Asn Leu Gly Glu Val Val Ser Asn Lys
   225                 230                 235                 240
   Pro Tyr Phe Phe Tyr Pro Pro Val Lys Pro Thr Gly Lys Thr Glu Val
                   245                 250                 255
   Asp Ile Arg Asn Ile Thr Ser Ile Pro Arg Val Asp Ile Leu Tyr Ser
               260                 265                 270
   Tyr Glu Asp Met His Asn Asp Thr Leu Tyr Ser Ala Ile Asp Asn Gly
           275                 280                 285
   Ala Lys Gly Ile Val Ile Ala Gly Ser Gly Ser Gly Ser Val Ser Thr
       290                 295                 300
   Pro Phe Ser Ala Ala Met Glu Asp Ile Thr Thr Lys His Asn Ile Pro
   305                 310                 315                 320
   Ile Val Ala Ser Thr Arg Thr Gly Asn Gly Glu Val Pro Ser Ser Ala
                   325                 330                 335
   Glu Ser Ser Gln Ile Ala Ser Gly Tyr Leu Asn Pro Ala Lys Ser Arg
               340                 345                 350
   Val Leu Leu Gly Leu Leu Leu Ala Gln Gly Lys Ser Ile Glu Glu Met
           355                 360                 365
   Arg Ala Val Phe Glu Arg Ile Gly Val Ala
       370                 375

## Description

### REFERENCE TO SEQUENCE LISTING

This application contains a Sequence Listing in computer readable form. The computer readable form is incorporated herein by reference.

### FIELD OF THE INVENTION

The present invention relates to stabilization of asparaginase enzymes.

### BACKGROUND OF THE INVENTION

It is well known that formation of acrylamide in heated food products can be reduced by reducing the amount of asparagine in the food materials, such as by subjecting the food materials to the action of the enzyme asparaginase (see, e.g., WO2004/026043 (The Procter & Gamble Company)).

WO 2006/053563 discloses processes for production of French fries which comprise contacting with asparaginase.

WO 2008/128974 discloses variants of asparaginase from *Aspergillus niger* having improved properties.

For some applications, to fit into the production line of specific food products, the asparaginase treatment should preferentially take place at a relatively high temperature. But asparaginase enzymes may not be stable at the industries' preferred temperatures to apply for the enzyme treatment. Also, asparaginase enzymes may not be stable even at lower temperatures, e.g., when being diluted or when being used continuously, such as for continuous or batch-wise dipping or incubation of potato pieces in asparaginase solution during the production of sliced potato chips or french fries.

It was therefore a purpose for the present inventors to find means of stabilizing asparaginase enzymes, especially at high temperatures or when being diluted or when being used continuously.

### SUMMARY OF THE INVENTION

The present inventors have surprisingly found that asparaginase enzymes are more stable at low pH. In particular, asparaginase enzymes are more thermostable at low pH. Therefore, the addition of an acid, such as sodium acid pyrophosphate, monopotassium phosphate or citric acid, to an aqueous solution of asparaginase improves the stability of the enzyme, such as its stability at high temperature, or its stability when being diluted.

Therefore, the invention relates to a method for producing par-fried french fries comprising:
a) contacting at a temperature of at least 42°C blanched potato strips with asparaginase at a pH which is lower than pH 6.5,
b) incubating the blanched potato strips for at least 5 minutes, and
c) processing the blanched potato strips to obtain the french fries, wherein the processing comprises par-frying,
wherein the contacting in step a) is performed by dipping and/or incubation in a solution or by spraying with a solution, said solution comprising (i) at least one acid selected among sodium acid pyrophosphate, monopotassium phosphate, citric acid, lactic acid, acetic acid, ascorbic acid, monocalcium phosphate and potassium bitartrate, and (ii) asparaginase at a concentration of 1,000-50,000 ASNU per litre, and
wherein the asparaginase is derived from *Aspergillus* and has at least 80% sequence identity to SEQ ID NO:1.

In a preferred embodiment, the contacting in step a) is performed by dipping or incubating the blanched potato strips into a solution comprising asparaginase. In an industrial process, such asparaginase solution will typically be reused, e.g., in a continuous process, and therefore enzyme stability is an extremely important parameter. Therefore, the method of the invention is particularly relevant in such processes, as declining asparaginase activity in the solution is presently a problem because of the industries' preferred high temperature, which may be further increased by continuous dipping/incubation of warm intermediate food products, combined with long usage time of the same solution and perhaps leakage of substance from the blanched potato strips. In such applications, an asparaginase solution having a low pH according to the method of the present invention is a huge advantage as it extends the stability of the asparaginase significantly.

### DETAILED DESCRIPTION OF THE INVENTION

### Asparaginase

An asparaginase according to the present invention may be an enzyme which is defined according to standard enzyme EC-classification as EC.3.5.1.1 and which catalyzes the following reaction:

L-asparagine + H₂O = L-aspartate + NH₄⁺

The asparaginase is derived from *Aspergillus.*

An asparaginase according to the present invention may have the amino acid sequence shown as SEQ ID NO:1 or an amino acid sequence having at least 80%, at least 90% or at least 95% identity to SEQ ID NO:1. Such an asparaginase may preferably be derived from *Aspergillus oryzae.*

For purposes of the present invention, the degree of identity between two amino acid sequences may be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment) An asparaginase according to the present invention may be a tetramer in its most active form.

### Method for producing a food product

As mentioned above, the invention relates to a method for producing
par-fried french fries comprising:
a) contacting at a temperature of at least 42°C blanched potato strips with asparaginase at a pH which is lower than pH 6.5,
b) incubating the blanched potato strips for at least 5 minutes, and
c) processing the blanched potato strips to obtain the french fries, wherein the processing comprises par-frying,
wherein the contacting in step a) is performed by dipping and/or incubation in a solution or by spraying with a solution, said solution comprising (i) at least one acid selected among sodium acid pyrophosphate, monopotassium phosphate, citric acid, lactic acid, acetic acid, ascorbic acid, monocalcium phosphate and potassium bitartrate, and (ii) asparaginase at a concentration of 1,000-50,000 ASNU per litre, and
wherein the asparaginase is derived from *Aspergillus* and has at least 80% sequence identity to SEQ ID NO:1.

Preferably, the incubation in step b) is performed for 7-15 minutes.

The contacting of the blanched potato strips with asparaginase at a pH which is lower than pH 6.5 may be performed by incubation or dipping of the blanched potato strips into a solution comprising asparaginase enzyme and having a pH which is lower than pH 6.5. In that case, the contacting in step a) and the incubating in step b) may be performed as one step, where the blanched potato strips are incubated in said solution for at least 5 minutes. Such incubation may be longer, e.g., at least 10 minutes, at least 15 minutes, at least 20 minutes or at least 30 minutes.

Alternatively, the dipping or incubation into the solution may be for a shorter time followed by drying of the blanched potato strips, e.g., in a drying oven. In that case, the drying step may be important for the asparaginase to work and therefore, if the dip is short, the incubation step b) in the method of the invention may be the drying of the intermediate form of the food product. Such short dipping or incubation into the solution may be, e.g., for between 2 seconds and 1 minute, such as for about 5 seconds, for about 10 seconds, for about 30 seconds or for about 45 seconds. Or the dipping may only be a very short dip with no incubation.

Alternatively, the contacting in step a) may be performed by spraying the blanched potato strips at a temperature of at least 42°C with a solution comprising asparaginase and having a pH which is lower than pH 6.5, followed by incubation, e.g., in a drying oven for at least 5 minutes, preferably for at least 10 minutes.

The drying may be performed, e.g., at an air temperature of above 60°C, preferably above 70°C, and more preferably of above 80°C, such as at a temperature of about 85-90°C. Alternatively, drying may be performed at lower temperature, e.g., at 40-45°C. Drying may also be performed at any other temperature, e.g., at 45-60°C. Drying may be performed, e.g., for between 5 and 30 minutes. Drying may be performed in an oven where temperature, humidity and/or air flow can be adjusted to the level(s) desired.

As mentioned above, the incubation step b) in a method of the invention may be the drying of the blanched potato strips. It may also be, though, that the blanched potato strips are incubated with the enzyme before optionally being dried. The incubation step b) may be any step in the manufacturing process of the blanched potato strips where the asparaginase enzyme is active and in contact with the blanched potato strips.

The solution comprising asparaginase and having a pH which is lower than pH 6.5 to be used in various embodiments of the method of the invention, i.e. the solution wherein the blanched potato strips are dipped and/or incubated or the solution which is sprayed onto the blanched potato strips, comprises asparaginase at a concentration of 1,000-50,000 ASNU per litre, preferably 2,000-20,000 ASNU per litre, more preferably 3,000-15,000 ASNU per litre and most preferably 4,000-10,000 ASNU per litre.

An asparaginase unit (ASNU) is defined as the amount of enzyme needed to generate 1.0 micromole of ammonia from hydrolysing asparagine in 1 minute at 37°C and pH 7.0. The concentration of asparagine when determining the activity may be 9.6 mg/ml.

The solution comprising asparaginase and having a pH which is lower than pH 6.5 to be used in various embodiments of the method of the invention, i.e. the solution wherein the intermediate form of the food product is dipped and/or incubated or the solution which is sprayed onto the intermediate form of the food product, comprises at least one acid selected among sodium acid pyrophosphate, monopotassium phosphate, citric acid, lactic acid, acetic acid, ascorbic acid, monocalcium phosphate and potassium bitartrate. Such acid or acids, optionally in combination with other acids, are to be present in the solution in sufficient amount so that the solution has a pH which is lower than pH 6.5. Alternatively, corresponding bases can be used and pH adjusted as desired using the relevant mixes of acids and corresponding salts or another acid.

The solution has a pH which is below pH 6.5. In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

Sodium acid pyrophosphate (Na₂H₂P₂O₇) is also sometimes referred to as SAPP or disodium pyrophosphate. Monopotassium phosphate (KH₂PO₄) is also sometimes referred to as potassium dihydrogen phosphate, KDP, or monobasic potassium phosphate, MKP. Potassium bitartrate (KC₄H₅O₆) is also sometimes referred to as potassium hydrogen tartrate or cream of tartar. Monocalcium phosphate (Ca(H₂PO₄)₂) is also sometimes referred to as calcium acid phosphate. In step c) in the method of the invention, the blanched potato strips are processed to obtain the par-fried French fries, wherein the processing comprises par-frying, optionally followed by freezing and packing. The final frying may be done by the consumer, e.g. at a restaurant or at home. The processing according to step c) may also comprise other steps.

In the preferred embodiment where the contacting in step a) is performed by dipping or incubating the blanched potato strips into a solution comprising asparaginase, enzyme stability is an extremely important parameter. In an industrial process, such enzyme solution will be reused. I.e., only a small fraction of the asparaginase will stick to the blanched potato strips after the dipping or incubation, whereas most of the asparaginase will remain in the solution. Thus the same enzyme solution may be used over a longer time period for the dipping or incubation of a large quantity of blanched potato strips for the production of french fries. In such process, the method of the present invention is particularly relevant, as declining asparaginase activity in the solution is presently a problem because of the industries' preferred high temperature, which is in part caused by a wish to avoid microbial growth, and which may be further increased by continuous dipping/incubation of warm blanched potato strips, combined with long usage time and perhaps leakage of substance from the blanched potato strips. In such applications, an asparaginase solution having a low pH according to the method of the present invention is a huge advantage as it extends the stability of the asparaginase significantly.

Therefore, in a more preferred embodiment, the contacting in step a) is performed by dipping or incubating the blanched potato strips into a solution comprising asparaginase and having a pH which is lower than pH 6.5, and the blanched potato strips are subsequently essentially separated from the asparaginase solution. In this context, essentially separated means that the blanched potato strips after the dipping/incubation retain at most 10%, such as 4-5%, (wt./wt.) sticking moisture. I.e., the weight of the blanched potato strips is increased by at most 10%, such as by 4-5%, because of asparaginase solution sticking to it after the dipping/incubation. Preferably according to this embodiment, the asparaginase solution is used for continuous or batch-wise dipping or incubation. Continuous or batch-wise dipping or incubation in this context means that the same asparaginase solution is used for the dipping or incubation of more than one batch of the blanched potato strips. Preferably, one litre of the asparaginase solution is used for the treatment of at least 2 kg potato substance, more preferably at least 5 kg, and even more preferably at least 10 kg.

In another more preferred embodiment, the contacting in step a) is performed by dipping or incubating the blanched potato strips into a solution comprising asparaginase and having a pH which is lower than pH 6.5, and the asparaginase solution is buffered such that pH is kept below 6.5 after the dipping or incubation of at least 2 kg, preferably at least 5 kg, more preferably at least 10 kg, of blanched potato strips per litre of asparaginase solution.

In the following, a solution comprising asparaginase and having a pH which is lower than pH 6.5 is sometimes referred to as an acidic asparaginase solution.

In a typical industrial production of french fries, potatoes are initially washed, sorted, steam peeled and cut. Following cutting, the potato strips are blanched in 2 to 3 sequential steps typically at 65-85°C for 10-30 min. Blanching is done to inactivate the endogenous enzymes in the potato, to partially cook the potato and to leach out reducing sugars to prevent excessive browning of the final product. After blanching, the potato strips may quickly be dipped, e.g. for 30-60 seconds, in a warm phosphate salt solution, e.g. a warm solution of sodium acid pyrophosphate, to prevent greying of the final product. The dip is optionally combined with a dip in glucose to control the final colour. The potatoes may be dried in a drier with hot circulating air at 75-95°C for 5-20 minutes giving a weight loss of 5-25%. Or they may be dried at a lower temperature, e.g., at 40-45°C, in which case the weight loss will be lower. Finally, the potato strips are par-fried before being quick-frozen and packed. Final frying is done at the restaurant or by consumers.

A french fry production method according to the present invention may be performed as described above, except that the intermediate form, i.e., the potato strips, are at some point before the par-frying contacted at a temperature of at least 42°C with an acidic asparaginase solution, such as a solution comprising asparaginase and an acid, preferably sodium acid pyrophosphate or monopotassium phosphate, and incubated for at least 5 minutes. Such contacting and incubation is performed following blanching. I.e., after blanching, the potato strips are contacted at a temperature of at least 42°C with an acidic asparaginase solution and incubated for at least 5 minutes. The contacting of the potato strips with the acidic asparaginase solution may be performed by incubation or dipping of the potato strips into a solution comprising asparaginase and, e.g., sodium acid pyrophosphate in one dip bath, and the incubation for at least 5 minutes may be performed either in the dip bath or in a following optional drying step, preferably for 7-15 min. Or the contacting may be performed by spraying the potato strips with an acidic asparaginase solution at a temperature of at least 42°C followed by incubation in a drier as described above for the typical industrial production of french fries. Following dipping/incubation in or spraying with the acidic asparaginase solution and optionally drying, the potato strips may be par-fried, quick-frozen and packed as described above. The potato strips may optionally be dipped in a glucose solution, e.g. before the contacting and incubation with the acidic asparaginase solution. Alternatively, glucose may be included in the acidic asparaginase solution, so that the potato strips are contacted with asparaginase, glucose and acid such as sodium acid pyrophosphate at the same time.

Also disclosed herein is a method for producing a heat-treated food product comprising:
a) contacting at a temperature of at least 42°C, preferably at least 45°C, at least 50°C or at least 55°C, more preferably at least 60°C, at least 62°C or at least 65°C, an intermediate form of said food product with asparaginase in the presence of an inorganic salt of phosphate or pyrophosphate,
b) incubating the intermediate form of the food product for at least 1 minute, and
c) processing the intermediate form of the food product to obtain the heat-treated food product,
wherein the processing comprises heating.

Preferably, the incubation in step b) is performed for at least 5 minutes.

In the context of the present disclosure, an inorganic salt of phosphate or pyrophosphate may be any inorganic salt of phosphate or pyrophosphate including any salt of PO₄³⁻, HPO₄²⁻, H₂PO₄⁻ and P₂O₇⁴⁻. Suitable examples may, e.g., be K₃PO₄, K₂HPO₄, KH₂PO₄, Na₃PO₄, Na₂HPO₄, NaH₂PO₄, (NH₄)₃PO₄, (NH₄)₂HPO₄, (NH₄)H₂PO₄, K₄P₂O₇, K₃HP₂O₇, K₂H₂P₂O₇, KH₃P₂O₇, Na₄P₂O₇, Na₃HP₂O₇, Na₂H₂P₂O₇, NaH₃P₂O₇, (NH₄)₄P₂O₇, (NH₄)₃HP₂O₇, (NH₄)₂H₂P₂O₇, (NH₄)H₃P₂O₇. However, this aspect of the disclosure is not limited to the salts of phosphate or pyrophosphate listed here.

In a preferred embodiment, the inorganic salt of phosphate or pyrophosphate is an acidic salt.

In a more preferred embodiment, the inorganic salt of phosphate or pyrophosphate is sodium acid pyrophosphate or monopotassium phosphate. In a more preferred embodiment, the inorganic salt of phosphate or pyrophosphate is sodium acid pyrophosphate.

In a preferred embodiment, the heat-treated food product is a vegetable-based food product. Preferably, such vegetable-based food product is derived from a vegetable tuber or root such as, but not limited to, the group consisting of potato, sweet potato, yams, yam bean, parsnip, parsley root, Jerusalem artichoke, carrot, radish, turnip, and cassava. A preferred food product produced by the method of the invention is a potato product. A more preferred food product produced by the method of the invention is french fries or sliced potato chips. Another more preferred food product produced by the method of the invention is a food product comprising potato granules or potato flakes, e.g. potato-based snacks.

Preferably, the inorganic salt of phosphate or pyrophosphate is present at a concentration which stabilizes the asparaginase. More preferably, the inorganic salt of phosphate or pyrophosphate is present at a concentration which stabilizes the asparaginase at high temperature, e.g. 42°C, 45°C, 50°C, 55°C, 60°C, 62°C or 65°C.

"Stabilizes the asparaginase" in the context of the present disclosure may mean that the half-life of the asparaginase is extended by at least a factor of 1.5, preferably by at least a factor of 2, more preferably by at least a factor of 3, and even more preferably by at least a factor of 5, when the inorganic salt of phosphate or pyrophosphate is present as compared to when it is not.

Half-life in the context of the present disclosure is the time after which the activity of the enzyme is reduced by 50% when being incubated under the relevant conditions. It may be determined as described in the Examples of the present application.

In a preferred embodiment, the contacting in step a) is performed by spraying a solution comprising asparaginase and an inorganic salt of phosphate or pyrophosphate onto the intermediate form of the food product.

In another preferred embodiment, the contacting in step a) is performed by dipping or incubating the intermediate form of the food product into a solution comprising asparaginase and an inorganic salt of phosphate or pyrophosphate.

In another preferred embodiment, the incubating in step b) is performed by drying the intermediate form of the food product at an air temperature of above 50°C.

In another preferred embodiment, the contacting in step a) and the incubating in step b) is performed as one step, where the intermediate form of the food product is incubated in a solution comprising (i) asparaginase and (ii) an inorganic salt of phosphate or pyrophosphate for at least 1 minute.

Preferably, such solution wherein the intermediate form of the food product is dipped and/or incubated, or which is sprayed onto the intermediate form of the food product, comprises asparaginase at a concentration of 1,000-50,000 ASNU per litre, more preferably 2,000-20,000 ASNU per litre, even more preferably 3,000-15,000 ASNU per litre and most preferably 4,000-10,000 ASNU per litre. Preferably, such solution comprises inorganic salt of phosphate or pyrophosphate, preferably sodium acid pyrophosphate or monopotassium phosphate, more preferably sodium acid pyrophosphate, at a concentration of 0.1-250 mM, more preferably 1-100 mM, even more preferably 5-50 mM.

Preferably, such solution wherein the intermediate form of the food product is dipped and/or incubated, or which is sprayed onto the intermediate form of the food product, has a pH which is lower than about pH 6.5, such as a pH which is below pH 6.5. In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

In a preferred embodiment, the food product is french fries or sliced potato chips.

In another preferred embodiment, the contacting in step a) is performed by blending asparaginase and an inorganic salt of phosphate or pyrophosphate into the intermediate form of the food product. Preferably, the concentration of asparaginase in the intermediate form of the food product is 100-10,000 ASNU per kg dry matter and the concentration of inorganic salt of phosphate or pyrophosphate is 0.05-5%.

Preferably, when the contacting in step a) is performed by blending asparaginase and an inorganic salt of phosphate or pyrophosphate into the intermediate form of the food product, the intermediate form of the food product has a pH which is lower than about pH 6.5, such as a pH which is below pH 6.5. In one preferred embodiment, the intermediate food product has a pH which is lower than about pH 6, such as a pH which is below pH 6. In another preferred embodiment, the intermediate form of the food product has a pH which is lower than about pH 5.8, such as a pH which is below pH 5.8. In another preferred embodiment, the intermediate form of the food product has a pH which is lower than about pH 5.7, such as a pH which is below pH 5.7. In another preferred embodiment, the solution has a pH of about pH 5.5.

In a preferred embodiment, the intermediate form of the food product comprises cooked mashed potatoes.

In some embodiments, the heating in step c) is performed by frying or par-frying. In other embodiments, the heating in step c) is performed by baking.

### Stabilized asparaginase composition at high temperature

Disclosed herein is also an aqueous composition having a temperature of at least 42°C, preferably at least 45°C, at least 50°C or at least 55°C, more preferably at least 60°C, at least 62°C or at least 65°C, which comprises (i) asparaginase and (ii) an inorganic salt of phosphate or pyrophosphate.

In one preferred embodiment, the inorganic salt of phosphate or pyrophosphate is an inorganic salt of pyrophosphate, preferably sodium acid pyrophosphate. In another preferred embodiment, the inorganic salt of phosphate or pyrophosphate is monopotassium phosphate or sodium acid pyrophosphate.

Preferably, the aqueous composition comprises an inorganic salt of phosphate or pyrophosphate, preferably sodium acid pyrophosphate or monopotassium phosphate, more preferably sodium acid pyrophosphate, at a concentration which stabilizes the asparaginase. More preferably, the inorganic salt of phosphate or pyrophosphate, preferably sodium acid pyrophosphate or monopotassium phosphate, more preferably sodium acid pyrophosphate, is present at a concentration which stabilizes the asparaginase at high temperature, e.g. 42°C, 45°C, 50°C, 55°C, 60°C, 62°C or 65°C.

In a preferred embodiment, the aqueous composition comprises asparaginase at a concentration of 1,000-50,000 ASNU per litre, preferably 2,000-20,000 ASNU per litre, more preferably 3,000-15,000 ASNU per litre and most preferably 4,000-10,000 ASNU per litre. In another preferred embodiment, the aqueous composition comprises an inorganic salt of phosphate or pyrophosphate, preferably sodium acid pyrophosphate or monopotassium phosphate, more preferably sodium acid pyrophosphate, at a concentration of 0.1-250 mM, preferably 1-100 mM, more preferably 5-50 mM.

Disclosed herein is also a method for making a heated aqueous composition, which comprises:
a) adding to water (i) asparaginase and (ii) an inorganic salt of phosphate or pyrophosphate, and
b) heating to a temperature of at least 42°C, preferably at least 45°C, at least 50°C or at least 55°C, more preferably at least 60°C, at least 62°C or at least 65°C,
wherein step a) is performed before, during or after step b).

It is to be understood that "adding to water" in step a) of such method does not necessarily mean adding to pure water. "Adding to water" includes adding to tap water with whatever ions, etc., are present in tap water. It also includes adding to any kind of water which has been purified or otherwise treated, e.g. deionised water, etc. It also includes adding to any aqueous solution which after step a) becomes an aqueous composition comprising asparaginase and an inorganic salt of phosphate or pyrophosphate.

The heating in step b) may be performed before adding the asparaginase and the inorganic salt of phosphate or pyrophosphate, it may be performed during the addition of asparaginase and the inorganic salt of phosphate or pyrophosphate, it may be performed after the addition of asparaginase and the inorganic salt of phosphate or pyrophosphate, it may be performed after the addition of the inorganic salt of phosphate or pyrophosphate but before addition of the asparaginase or it may be performed after the addition of asparaginase but before addition of the inorganic salt of phosphate or pyrophosphate, although this last option is less preferable as the enzyme may be inactivated in the heated water before the addition of phosphate/pyrophosphate. The heating of the water need not take place in the tank where the asparaginase and the inorganic salt of phosphate or pyrophosphate are applied. The water may be heated before filling it into the tank.

The asparaginase may be added before, at the same time or after the addition of the inorganic salt of phosphate or pyrophosphate. Preferably, the asparaginase is added after the addition of the inorganic salt of phosphate or pyrophosphate.

In one preferred embodiment, the inorganic salt of phosphate or pyrophosphate is an inorganic salt of pyrophosphate, preferably sodium acid pyrophosphate. In another preferred embodiment, the inorganic salt of phosphate or pyrophosphate is monopotassium phosphate or sodium acid pyrophosphate.

Preferably, the inorganic salt of phosphate or pyrophosphate is added at a concentration which stabilizes the asparaginase. More preferably, the inorganic salt of phosphate or pyrophosphate is added at a concentration which stabilizes the asparaginase at high temperature, e.g. 42°C, 45°C, 50°C, 55°C, 60°C, 62°C or 65°C.

Preferably, asparaginase is added at a concentration of 1,000-50,000 ASNU per litre, more preferably 2,000-20,000 ASNU per litre, even more preferably 3,000-15,000 ASNU per litre and most preferably 4,000-10,000 ASNU per litre. Preferably, the inorganic salt of phosphate or pyrophosphate is added at a concentration of 0.1-250 mM, more preferably 1-100 mM, and even more preferably 5-50 mM.

A heated asparaginase composition wherein the asparaginase has been stabilized with, e.g., monopotassium phosphate or sodium acid pyrophosphate, or with another acid, such as, e.g., citric acid, lactic acid, acetic acid, ascorbic acid, monocalcium phosphate or potassium bitartrate, may be useful, e.g., in the food industry, where, for various reasons, asparaginase treatment of food products before heating of these should preferentially take place at a relatively high temperature to fit into an existing production line. In such processes, the asparaginase may be unstable especially in the start-up phase of a continuous process. When the asparaginase is added to fairly pure tap water, which is optionally heated, its activity may be reduced, in which case the present inventors have surprisingly found that lowering the pH stabilizes the enzyme and gives a higher activity in an aqueous solution, especially in an aqueous solution having a high temperature.

When the continuous process is running more or less in a steady-state, for example a continuous process of french fries where potato strips are dipped continuously in a hot solution of asparaginase, leakage of solubles from the potato strips may contribute to stabilization of the asparaginase enzyme and therefore even though further addition of, e.g., enzyme and water may be needed along the continuous process to maintain a steady-state level, the level of acid may be allowed to be reduced to a lower level at this stage as compared to the start-up phase.

Therefore, disclosed herein is a method for starting up a continuous industrial process, which comprises:
a) making an aqueous solution of asparaginase having a pH which is lower than about pH 6.5, and
b) heating to a temperature of at least 42°C, preferably at least 45°C, at least 50°C or at least 55°C, more preferably at least 60°C, at least 62°C or at least 65°C,
wherein step a) is performed before, during or after step b).

In a preferred embodiment, the continuous industrial process is a process for producing par-fried french fries where potato strips are continuously dipped or incubated in a solution comprising asparaginase.

Preferably, also for this disclosure, asparaginase is added at a concentration of 1,000-50,000 ASNU per litre, more preferably 2,000-20,000 ASNU per litre, even more preferably 3,000-15,000 ASNU per litre and most preferably 4,000-10,000 ASNU per litre.

Preferably, also for this disclosure, the aqueous asparaginase solution having a pH which is lower than about pH 6.5 comprises at least one acid selected among sodium acid pyrophosphate, monopotassium phosphate, citric acid, lactic acid, acetic acid, ascorbic acid, monocalcium phosphate and potassium bitartrate. Such acid or acids, optionally in combination with other acids, are to be present in the solution in sufficient amount so that the solution has a pH which is lower than about pH 6.5. Preferably, the solution has a pH which is below pH 6.5. In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

### Concentrated asparaginase composition

Disclosed herein is also a concentrated composition of asparaginase comprising (i) asparaginase at a concentration of at least 100 ASNU per gram and (ii) an inorganic salt of phosphate or pyrophosphate at a concentration of at least 0.1 mM, preferably at least 1 mM, more preferably at least 5 mM.

Stabilization of asparaginase may be advantageous already at the stage of its production. I.e., an inorganic salt of phosphate or pyrophosphate, preferably an acidic inorganic salt of phosphate or pyrophosphate, such as sodium acid pyrophosphate or monopotassium phosphate, may be added during fermentation of a microorganism expressing asparaginase. Or it may be added after fermentation to the fermentation broth comprising the asparaginase.

Optionally, the asparaginase may be purified. In that case, a stabilizing compound such as an acid, e.g., sodium acid pyrophosphate or monopotassium phosphate, may be added to the asparaginase before or after it has been purified.

The term "purified" as used herein covers asparaginase preparations where the preparation has been enriched for the asparaginase enzyme. Such enrichment could for instance be: the removal of the cells of the organism from which the asparaginase was produced, the removal of non-protein material by a protein specific precipitation or the use of a chromatographic procedure where the asparaginase is selectively adsorbed and eluted from a chromatographic matrix. The asparaginase may have been purified to an extent so that only minor amounts of other proteins are present. The expression "other proteins" relate in particular to other enzymes.

Therefore disclosed herein is also an aqueous composition comprising (i) asparaginase at a concentration of at least 100 ASNU per gram and (ii) an inorganic salt of phosphate or pyrophosphate, preferably an acidic inorganic salt of phosphate or pyrophosphate, such as sodium acid pyrophosphate or monopotassium phosphate, at a concentration of at least 0.1 mM, preferably at least 1 mM, more preferably at least 5 mM.

Such aqueous composition of asparaginase which has been stabilized, e.g., with sodium acid pyrophosphate or monopotassium phosphate according to the present invention comprises asparaginase at a concentration of at least 100 ASNU per gram, preferably at least 1000 or at least 2000 ASNU per gram, more preferably at least 3000 ASNU per gram, such as about 3500 ASNU per gram.

In a preferred embodiment, such aqueous composition comprising asparaginase and an inorganic salt of phosphate or pyrophosphate has been formulated to further stabilize the asparaginase. In another preferred embodiment, such aqueous composition comprising asparaginase and an inorganic salt of phosphate or pyrophosphate comprises glycerol, preferably at a concentration of 40-60%.

Such aqueous composition comprising asparaginase and an inorganic salt of phosphate or pyrophosphate may be stored as a liquid composition until the asparaginase is to be applied by industry, e.g. for industrial production of a food product, such as a heat-treated food product.

Alternatively, an aqueous asparaginase composition comprising a stabilizing compound such as an acid, e.g., sodium acid pyrophosphate or monopotassium phosphate, may be formulated into an enzyme granulate, e.g. by spray-drying.

Therefore disclosed herein is also an enzyme granulate comprising (i) asparaginase at a concentration of at least 100 ASNU per gram and (ii) an inorganic salt of phosphate or pyrophosphate, such as sodium acid pyrophosphate or monopotassium phosphate, at a concentration of at least 0.1 mM, preferably at least 1 mM, more preferably at least 5 mM . The term "enzyme granulate" as used herein is a granular formulation of the asparaginase enzyme. The enzyme granulate may also comprise other enzymes.

Disclosed herein is also an enzyme granulate comprising asparaginase at a concentration of at least 100 ASNU per gram and having a pH which is lower than about pH 6.

In the context of the present disclosure, the pH of an enzyme granulate means the pH of an aqueous solution or slurry of such enzyme granulate in deionised water.

Such enzyme granulate may comprise at least one acid selected among sodium acid pyrophosphate, monopotassium phosphate, citric acid, lactic acid, acetic acid, ascorbic acid, monocalcium phosphate and potassium bitartrate.

An enzyme granulate of the present disclosure comprises asparaginase at a concentration of at least 100 ASNU per gram, preferably at least 1000 or at least 2000 ASNU per gram, more preferably at least 3000 ASNU per gram, such as about 3500 ASNU per gram.

Disclosed herein is also a method for producing an enzyme granulate comprising asparaginase, which method comprises:
a) adding an inorganic salt of phosphate or pyrophosphate, such as sodium acid pyrophosphate or monopotassium phosphate, to an aqueous solution comprising asparaginase, and
b) formulating said aqueous solution into an enzyme granulate.

Preferably, the inorganic salt of phosphate or pyrophosphate is added at a concentration of at least 0.1 mM, more preferably at least 1 mM, and even more preferably at least 5 mM.

Disclosed herein is also a method for producing an enzyme granulate comprising asparaginase, which method comprises:
a) making an aqueous solution comprising asparaginase and having a pH which is lower than about pH 6, and
b) formulating said aqueous solution into an enzyme granulate.

Preferably, the aqueous solution has a pH which is below pH 6. In one preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

Preferably, following step a), the asparaginase is allowed to "age" in the acidic solution, e.g. for 0.5 to 10 hours, before the solution is formulated into an enzyme granulate. Without wishing to be bound by any theory, it may be that the enzyme is more active in a multimeric form, e.g. as a dimer or a tetramer, and such aging may allow the multimers to assemble thus rendering the enzyme granulate more active and more stable.

Sodium chloride, NaCl, is often added to purified enzyme liquid before granulation. However, NaCl may destroy the activity of the asparaginase enzyme. Therefore, the methods of the present invention of making a stabilized asparaginase granulate may be especially relevant in the situation where the asparaginase granulate also comprises NaCl.

### Stock solution of asparaginase

Stabilization of asparaginase according to the present disclosure may be advantageous at the stage where the concentrated formulated composition of asparaginase is to be applied into an industrial process, e.g. for production of a heat treated food product. At this stage, the industrial food processors in many cases wish to make a stock solution of the enzyme having a suitable concentration to apply into their production line. However, such stock solution of asparaginase may lose its activity when being stored, especially at elevated temperatures, such as at room temperature or higher.

The present inventors have surprisingly found that lowering the pH stabilizes the asparaginase in such stock solution.

For example, in production of dough-based biscuits or snacks, asparaginase will typically be added with the liquid and mixed into the dry ingredients or added directly to the dry ingredients before mixing of the dough. However, to facilitate correct dosing, it might be optimal to have an enzyme stock solution of lower activity than the formulated, concentrated enzyme product. To ensure enzyme stability of this intermediate stock solution during processing in potentially warm industrial facilities an acid may be added according to the present invention.

Therefore, disclosed herein is also an aqueous stock solution comprising asparaginase at a concentration of 100-2,000 ASNU per gram and having a pH which is lower than about pH 6.5.

Preferably, the aqueous stock solution has a pH which is below pH 6.5. In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

Disclosed herein is also a method for diluting or dissolving a concentrated composition of asparaginase for applying it into an industrial process, which comprises:
a) diluting or dissolving a concentrated composition comprising asparaginase at a concentration of at least 2,000 ASNU per gram into a stock solution comprising less than half of the asparaginase activity per gram as compared to the concentrated composition and having a pH which is lower than about pH 6.5, and
b) applying the stock solution into the industrial process.

The concentrated composition comprising asparaginase may be an aqueous composition, in which case it is diluted into a stock solution, or it may be an enzyme granulate, in which case it is dissolved into a stock solution.

Preferably, also in this disclosure, the aqueous stock solution has a pH which is below pH 6.5.

In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

In a preferred embodiment, the stock solution is diluted by at least a factor of 2 (w/w) when applying it into the industrial process. In a more preferred aspect, the stock solution is diluted by at least a factor of 5 (w/w), more preferably by at least a factor of 10 (w/w), when applying it into the industrial process.

Preferably, at least a part of the stock solution is stored for at least 6 hours before applying it into the industrial process.

Preferably, in step a), the concentrated composition comprising asparaginase is diluted or dissolved into a stock solution comprising less than 20%, more preferably less than 10%, of the asparaginase activity per gram as compared to the concentrated composition.

Disclosed herein is also a method for diluting or dissolving a concentrated composition of asparaginase for applying it into a dough, which comprises:
a) diluting or dissolving a concentrated composition comprising asparaginase at a concentration of at least 2,000 ASNU per gram into a stock solution comprising less than half of the asparaginase activity per gram as compared to the concentrated composition and having a pH which is lower than about pH 6.5, and
b) applying the stock solution into the dough.

Preferably, the dough is to be heat-treated, such as by baking or by frying.

Preferably, also in this disclosure, the stock solution has a pH which is below pH 6.5. In one preferred embodiment, the solution has a pH which is lower than about pH 6, such as a pH which is below pH 6. In another preferred embodiment, the solution has a pH which is lower than about pH 5.8, such as a pH which is below pH 5.8. In another preferred embodiment, the solution has a pH which is lower than about pH 5.7, such as a pH which is below pH 5.7. In another preferred embodiment, the solution has a pH of about pH 5.5.

In a preferred aspect, the stock solution is diluted by at least a factor of 2 (w/w) when applying it into the dough. In a more preferred aspect, the stock solution is diluted by at least a factor of 5 (w/w), more preferably by at least a factor of 10 (w/w), when applying it into the dough.

Preferably, at least a part of the stock solution is stored for at least 6 hours before applying it into the dough.

Preferably, in step a), the concentrated composition comprising asparaginase is diluted or dissolved into a stock solution comprising less than 20%, more preferably less than 10%, of the asparaginase activity per gram as compared to the concentrated composition.

Disclosed herein is also an aqueous stock solution of asparaginase comprising (i) asparaginase at a concentration of at least 100 ASNU per gram and (ii) an inorganic salt of phosphate or pyrophosphate, preferably sodium acid pyrophosphate or monopotassium phosphate, at a concentration of at least 0.1 mM. In a preferred aspect, such stock solution comprises asparaginase at a concentration of 100-2,000 ASNU per gram. In another preferred aspect, such stock solution comprises an inorganic salt of phosphate or pyrophosphate at a concentration of at least 1 mM, preferably at least 5 mM.

Disclosed herein is also a method for diluting or dissolving a concentrated composition of asparaginase for applying it into an industrial process, which comprises:
a) diluting or dissolving a concentrated composition comprising asparaginase at a concentration of at least 2,000 ASNU per gram into a stock solution comprising less than half of the asparaginase activity per gram as compared to the concentrated composition and further comprising at least 0.1 mM, preferably at least 1 mM, more preferably at least 5 mM of an inorganic salt of phosphate or pyrophosphate, and
b) applying the stock solution into the industrial process.

Disclosed herein is also a method for diluting or dissolving a concentrated composition of asparaginase for applying it into a dough, which comprises:
a) diluting or dissolving a concentrated composition comprising asparaginase at a concentration of at least 2,000 ASNU per gram into a stock solution comprising less than half of the asparaginase activity per gram as compared to the concentrated composition and further comprising at least 0.1 mM, preferably at least 1 mM, more preferably at least 5 mM of an inorganic salt of phosphate or pyrophosphate, and
b) applying the stock solution into the dough.

### EXAMPLES

### METHODS AND MATERIALS (EXAMPLES 1-8)

### ENZYME STABILITY IN WATER OF DIFFERENT QUALITY, IN SOLUTIONS CONTAINING SAPP, KH₂PO₄, AND GLUCOSE

### ASNU MICROTITER ASSAY (as used in Examples 1-8)

Enzyme activity was determined in a buffer assay at 37°C, pH 7.0 using asparagine as substrate. 1 ASNU is defined as the amount of enzyme releasing one micromole of ammonium from hydrolysis of asparagine per minute at the above conditions.

The produced ammonium is combined with alpha-ketoglutarate to form glutamic acid, whereby NADH is oxidised to NAD⁺. The consumption of NADH is measured with time at 340 nm. Activity is determined relative to known standards.

### Reagents:

### MOPS Buffer:

0.1 M MOPS, pH 7.00, 0.01% Triton X-100
Adjust pH to 7.00 +/-0.05 using 4M NaOH before adding Triton X-100

### Reagent A:

10 mg/ml L-Asparagine in MOPS buffer
0.44 mg/ml NADH
2.52 mg/ml alpha-ketoglutarate
2.24 mg/ml GIDH (>65 Units/ml)

Weigh out L-asparagine and dissolve in MOPS buffer.

Weigh out NADH, alpha-ketoglutarate and GIDH and dissolve in MOPS buffer.

Mix the two solutions and fill to volume using MOPS buffer.

### Standards:

0.5 - 1.0 - 1.5 - 1.9 - 2.5 - 3.0 - 3.4 ASNU/ml.

### Samples:

Unknown samples are diluted in MOPS buffer to approximately 1-3 ASNU/g.

### Procedure:

Use a 96 well microtitter plate

Adjust temperature in the ELISA reader so that the liquid temperature in the wells is 37°C.

240 µL reagent A is added to each well and the plate prehetated for 10 min.

10 µL sample or standard is added

After 30 sec. standing, the plate is shaken for 30 sec. and measurement started at 340 nm.

Measurements are recorded every 10 sec. for a total time of 6 min. (readings started 1 min. after enzyme addition). Blind samples are run using 10 µL MOPS buffer instead of sample.

Absorbance (OD340) vs. time is depicted for each standard and sample and the slope (decay) calculated based on data from readings at t=4 min to t=6 min. The slope represents the drop in NADH which is directly proportional to the NH₄⁺ produced by the asparaginase action.

A standard curve based on the calculated slopes is made showing the slope vs. enzyme activity in ASNU/ml. Activity in the unknown samples are determined from the calculated slope by comparison to the standard curve.

### THERMOSTABILITY ASSAY (as used in Examples 1-8)

### Procedure:

Enzyme was incubated in the relevant solution at a dosage of 10 ASNU/g, and a temperature of 25, 55, and 60°C unless otherwise stated. The 25°C sample was made as a control ensuring that enzyme activity was not altered significantly in the presence of the compounds tested.

Samples were taken out right after enzyme addition and mixing (30 sec.) and subsequently after 30, 60, 90, 120, and 150 min. and held on ice until activity analysis.

Enzyme activity measured in the solution (ASNU/ml) at the different conditions is shown in the following. Enzyme stability is given as the estimated half-life using a computed best fit exponential decay curve, i.e. E = E0* exp(-ln(2)/T½*t), where E0 = enzyme activity at time 0, T½ = half-life equivalent to the time where activity is 50% of initial, and t = time.

### EXAMPLE 1

*Stability in deionised water*

| | ASNU/ml | | |
|---|---|---|---|
| Time (min) | 25°C | 55°C | 60°C |
| 0.5 | 9.6 | 9.4 | 8.9 |
| 30 | 9.5 | 9.2 | 6.0 |
| 60 | 9.4 | 8.7 | 4.4 |
| 90 | 8.1 | 8.2 | 3.4 |
| 120 | 10.1 | 8.2 | 2.6 |
| 150 | 8.4 | 7.7 | 2.1 |

Enzyme stability in deionised water is very good at 55°C showing an estimated half-life of 535 min. At 60°C, stability is lower with a half-life of 70 min.

### EXAMPLE 2

*Stability in tapwater*

| | ASNU/ml | | |
|---|---|---|---|
| Time (min) | 25°C | 55°C | 60°C |
| 0.5 | 9.2 | 0.08 | 0.09 |
| 30 | 8.7 | 0.11 | 0.11 |
| 60 | 8.6 | 0.10 | 0.07 |
| 90 | 8.5 | 0.09 | 0.08 |
| 120 | 8.3 | 0.05 | 0.06 |
| 150 | 8.0 | 0.06 | 0.05 |

Enzyme stability in tap water is very poor. Practically no activity at all is detected at 55 and 60°C, even in samples taken after only 0.5 min. Activity at 25°C show a slight drop with time.

### EXAMPLE 3

*Stability in tapwater (25°C, 40°C and 50°C)*

| | ASNU/ml | | |
|---|---|---|---|
| Time (min) | 25°C | 40°C | 50°C |
| 0.5 | 8.52 | 7.92 | 0.39 |
| 30 | 7.72 | 2.43 | 0.09 |
| 60 | 7.44 | 0.96 | 0.09 |
| 90 | 7.64 | 0.49 | 0.08 |
| 120 | 7.06 | 0.33 | 0.08 |
| 150 | 6.74 | 0.26 | 0.08 |

At lower temperature, enzyme stability in tap water is improved slightly. At 40°C some activity can be detected until 60 min. and estimated half-life is approximately 20 min, while little if any activity is detected at 50°C. Activity at 25°C shows a slight drop with time.

### EXAMPLE 4

*Stability in deionised water with 22.5 mM SAPP (0.5%)*

| | ASNU/ml | | |
|---|---|---|---|
| Time (min) | 25°C | 55°C | 60°C |
| 0.5 | 9.36 | 9.80 | 9.69 |
| 30 | 8.95 | 9.33 | 8.67 |
| 60 | 8.35 | 9.38 | 7.93 |
| 90 | 8.78 | 8.94 | 7.52 |
| 120 | 8.59 | 9.22 | 7.71 |
| 150 | 8.52 | 9.20 | 7.14 |

Addition of 22.5 mM Sodium Acid Pyrophosphate (SAPP) to deionised water increases enzyme stability at 60°C significantly. Half-life is estimated to be 375 min. compared to the 70 min. observed in deionised water. At 55°C half-life also increased to approximately 1800 min.

### EXAMPLE 5

*Stability in deionised water with 4.5 mM SAPP* (*0*.*1*%*)*

| | ASNU/ml | | |
|---|---|---|---|
| Time (min) | 25°C | 55°C | 60°C |
| 0.5 | 8.87 | 9.01 | 8.70 |
| 30 | 8.46 | 8.80 | 7.61 |
| 60 | 8.70 | 8.72 | 7.04 |
| 90 | 8.84 | 8.60 | 6.69 |
| 120 | 8.62 | 8.50 | 6.23 |
| 150 | 8.67 | 8.38 | 5.95 |

Increases in stability were also observed when the amount of SAPP used was reduced to 4.5 mM. Half-life at 60°C was approximately 285 min. and at 55°C approximately 1520 min.

### EXAMPLE 6

*Stability in tapwater with 22.5 mM SAPP (0.5%)*

| | ASNU/ml | | |
|---|---|---|---|
| Time (min) | 25°C | 55°C | 60°C |
| 0.5 | 9.50 | 10.36 | 9.61 |
| 30 | 9.00 | 9.43 | 8.09 |
| 60 | 8.63 | 9.59 | 7.59 |
| 90 | 8.73 | 9.31 | 6.96 |
| 120 | 8.79 | 9.49 | 6.88 |
| 150 | 8.66 | 9.55 | 6.60 |

Addition of SAPP also increased enzyme stability significantly in tapwater. Half-life at 60°C rose to 297 min. compared to activity being non-detectable in tapwater without SAPP. At 55°C half-life increased to 1735 min. again from a non-detectable level in tapwater.

### EXAMPLE 7

*Stability in deionised water with 50 mM KH₂PO₄, pH = 5*

| | ASNU/ml | |
|---|---|---|
| Time (min) | 25°C | 60°C |
| 0.5 | 9.85 | 8.19 |
| 60 | 9.93 | 7.75 |
| 120 | 9.41 | 7.31 |
| 150 | 9.73 | 7.12 |

Also phosphate (50 mM) stabilises the enzyme significantly at 60°C.

### EXAMPLE 8

*Stability in deionised water with 1% glucose*

| | ASNU/ml | | |
|---|---|---|---|
| Time (min) | 25°C | 55°C | 60°C |
| 0.5 | 9.53 | 10.14 | 9.48 |
| 30 | 8.99 | 10.15 | 6.09 |
| 60 | 9.10 | 9.46 | 4.73 |
| 90 | 9.29 | 9.36 | 3.74 |
| 120 | 9.95 | 9.16 | 2.94 |
| 150 | 9.12 | 9.37 | 2.46 |

Addition of extra dry matter in the form of 1% glucose did not affect enzyme stability in deionised water. Estimated half-life at 55°C is 1015 min and at 60°C it is 60 min.

### METHODS AND MATERIALS (EXAMPLES 9-11)

### ENZYME STABILITY IN SOLUTIONS CONTAINING SAPP OR CITRIC ACID AT DIFFERENT PH

### ASNU ASSAY (as used in Examples 9-11)

Enzyme activity was determined in a buffer assay at 37°C, pH 7.0 using asparagine as substrate. 1 ASNU is defined as the amount of enzyme releasing one micromole of ammonium from hydrolysis of asparagine per minute at the above conditions. The produced ammonium is quantified using Nesslers reagent. Absorbance is measured at 436 nm. Activity is determined relative to known standards.

### Reagents

### Tris Buffer

35 mM Tris, pH 7,00, 0,01% Triton X-100

### Asparagine

25 mg/ml L-Asparagine in Tris buffer

### TCA

1.5 M Trichloroacetic acid

### Standards

0 - 2 - 4 - 8 - 10 ASNU/ml.

### Procedure

### Activity assay

Mix 3.75 ml buffer and 0.5 ml asparagine solution in a glass tube and preheat in a water bath at 37 °C for 5-10 min. Add 0.5 ml standard or sample and incubate at 37°C for 10 min. Add 0.25 ml TCA to stop the reaction.

### Ammonia determination

Take out 0.05 ml of the solution above; add 0.85 ml MQ water, 0.1 ml Nesslers reagent and mix. After 10 min the absorbance is read in a spectrophotometer at 436 nm.

A standard curve showing absorbance vs. enzyme concentration in the standards is made and activities in the unknown samples calculated from this.

### THERMOSTABILITY ASSAY (as used in Examples 9-11)

### Procedure

Enzyme was incubated in the relevant solution at a concentration of 10 ASNU/ml, and a temperature of 25 and 60°C unless otherwise stated. The 25°C sample was made as a control ensuring that enzyme activity was not altered significantly in the presence of the compounds tested.

For initial screening of enzyme stability, samples were taken out 5 min after enzyme addition and held on ice until activity analysis. For determination of enzyme half-life (T½) samples were taken out after 5, 40, 80, 120 min.

Enzyme activity measured in the solution at the different conditions is shown in the following. Enzyme stability is given as the estimated half-life using a computed best fit exponential decay curve, i.e. E = E0* exp(-ln(2)/T½*t), where E0 = enzyme activity at time 0, T½ = half-life equivalent to the time where activity is 50% of initial, and t = time.

### EXAMPLE 9

*Activity after 5 min holding in 0.5% SAPP in deionised water at different pH*

| pH | 25°C | 60°C |
|---|---|---|
| 5 | 9.8 | 9.0 |
| 6 | 9.6 | 7.0 |
| 7 | 9.4 | 0.7 |
| 8 | 8.8 | 0.7 |

Enzyme stability at 60°C in 0.5% SAPP in deionised water is clearly dependent upon pH. At 25°C enzyme activity drops slightly after 5 min holding at pH > 7, while at 60°C nearly all activity is lost after 5 min holding at pH >6.

### EXAMPLE 10

*Stability in 0.5% SAPP in deionised water, pH 5*

| | ASNU/ml | | | |
|---|---|---|---|---|
| Time (min) | 25°C | 50°C | 60°C | 65°C |
| 5 | 10.6 | 10.6 | 9.9 | 5.7 |
| 40 | | 10.4 | 9.1 | 0 |
| 80 | | 9.7 | 8.7 | 0 |
| 120 | 10.2 | 9.5 | 8.5 | 0 |

Enzyme is stable for more than 120 min when held at a temperature of maximum 60°C and pH 5. Estimated half-life is 8 hrs. At 65°C activity drops very fast, showing no activity at all after 40 min.

### EXAMPLE 11

*Stability in 20 mM citric acid in deionised water, pH 5*

| | ASNU/ml | | |
|---|---|---|---|
| Time (min) | 25°C | 60°C | 65°C |
| 5 | 9.4 | 11.0 | 10.8 |
| 40 | | 10.9 | 8.9 |
| 80 | | 10.8 | 8.1 |
| 120 | 10.7 | 10.5 | 7.4 |

In the presence of citric acid, pH 5, the enzyme is stable also at 65°C, having an estimated half-life of approximately 3 hrs.

## Claims

1. A method for producing par-fried french fries comprising:
a) contacting at a temperature of at least 42°C blanched potato strips with asparaginase at a pH which is lower than pH 6.5,
b) incubating the blanched potato strips for at least 5 minutes, and
c) processing the blanched potato strips to obtain the french fries, wherein the processing comprises par-frying,
wherein the contacting in step a) is performed by dipping and/or incubation in a solution or by spraying with a solution, said solution comprising (i) at least one acid selected among sodium acid pyrophosphate, monopotassium phosphate, citric acid, lactic acid, acetic acid, ascorbic acid, monocalcium phosphate and potassium bitartrate, and (ii) asparaginase at a concentration of 1,000-50,000 ASNU per litre, and
wherein the asparaginase is derived from *Aspergillus* and has at least 80% sequence identity to SEQ ID NO:1.

2. The method according to claim 1, wherein the contacting in step a) is performed by spraying a solution comprising asparaginase and having a pH which is lower than pH 6.5 onto the intermediate form of the food product.

3. The method according to claims 1, wherein the contacting in step a) is performed by dipping or incubating the intermediate form of the food product into a solution comprising asparaginase and having a pH which is lower than pH 6.5.

4. The method according to any of claims 2-3, wherein the incubating in step b) is performed by drying the intermediate form of the food product at an air temperature of above 50°C.

5. The method according to claim 3, wherein the contacting in step a) and the incubating in step b) is performed as one step, where the intermediate form of the food product is incubated in the solution for at least 5 minutes.

## Patentansprüche

1. Verfahren zum Herstellen vorfrittierter Pommes frites, umfassend:
a) bei einer Temperatur von wenigstens 42 °C Inkontaktbringen von blanchierten Kartoffelstreifen mit Asparaginase bei einem pH-Wert, der niedriger als pH 6,5 ist,
b) Inkubieren der blanchierten Kartoffelstreifen für wenigstens 5 Minuten und
c) Verarbeiten der blanchierten Kartoffelstreifen, um die Pommes frites zu erhalten, wobei die Verarbeitung Vorfrittieren umfasst,
wobei das Inkontaktbringen bei Schritt a) durch Eintauchen und/oder Inkubieren in einer Lösung oder durch Besprühen mit einer Lösung durchgeführt wird, wobei die Lösung (i) wenigstens eine Säure ausgewählt aus saurem Natriumpyrophosphat, Monokaliumphosphat, Citronensäure, Milchsäure, Essigsäure, Ascorbinsäure, Monocalciumphosphat und Kaliumbitartrat und (ii) Asparaginase mit einer Konzentration von 1.000-50.000 ASNU pro Liter umfasst, und
wobei die Asparaginase aus *Aspergillus* abgeleitet ist und wenigstens 80 % Sequenzidentität mit SEQ ID NO:1 aufweist.

2. Verfahren gemäß Anspruch 1, wobei das Inkontaktbringen bei Schritt a) durch Sprühen einer Lösung, die Asparaginase umfasst und einen pH-Wert, der niedriger als pH 6,5 ist, aufweist, auf die Zwischenform des Lebensmittelprodukts durchgeführt wird.

3. Verfahren gemäß Anspruch 1, wobei das Inkontaktbringen bei Schritt a) durch Eintauchen oder Inkubieren der Zwischenform des Lebensmittelprodukts in eine Lösung, die Asparaginase umfasst und einen pH-Wert, der niedriger als pH 6,5 ist, aufweist, durchgeführt wird.

4. Verfahren gemäß einem der Ansprüche 2-3, wobei das Inkubieren bei Schritt b) durch Trocknen der Zwischenform des Lebensmittelprodukts bei einer Lufttemperatur von über 50 °C durchgeführt wird.

5. Verfahren gemäß Anspruch 3, wobei das Inkontaktbringen bei Schritt a) und das Inkubieren bei Schritt b) als ein Schritt durchgeführt wird, wobei die Zwischenform des Lebensmittelprodukts wenigstens 5 Minuten in der Lösung inkubiert wird.

## Revendications

1. Procédé de production de pommes de terres frites préfrites comprenant :
a) la mise en contact à une température d'au moins 42 °C de bâtonnets de pommes de terre blanchis avec de l'asparaginase à un pH qui est inférieur à pH 6,5,
b) l'incubation des bâtonnets de pommes de terre blanchis pendant au moins 5 minutes, et
c) le traitement des bâtonnets de pommes de terre blanchis pour obtenir les pommes de terre frite, le traitement comprenant une préfriture,
dans lequel la mise en contact dans l'étape a) est conduit par immersion et/ou incubation dans une solution ou par pulvérisation avec une solution, ladite solution comprenant (i) au moins un acide choisi parmi le pyrophosphate acide de sodium, le phosphate monopotassique, l'acide citrique, l'acide lactique, l'acide acétique, l'acide ascorbique, le phosphate monocalcique et le bitartrate de potassium, et (ii) de l'asparaginase à une concentration de 1 000 à 50 000 ASNU par litre, et
dans lequel l'asparaginase est dérivée d'*Aspergillus* et présente au moins 80 % d'identité de séquence avec SEQ ID NO: 1.

2. Procédé selon la revendication 1, dans lequel la mise en contact dans l'étape a) est conduite par pulvérisation d'une solution comprenant de l'asparaginase et ayant un pH qui est inférieur à pH 6,5 sur la forme intermédiaire du produit alimentaire.

3. Procédé selon la revendication 1, dans lequel la mise en contact dans l'étape a) est conduite par immersion ou incubation de la forme intermédiaire du produit alimentaire dans une solution comprenant de l'asparaginase et ayant un pH qui est inférieur à pH 6,5.

4. Procédé selon l'une quelconque des revendications 2 à 3, dans lequel l'incubation dans l'étape b) est conduite par séchage de la forme intermédiaire du produit alimentaire à une température d'air supérieure à 50 °C.

5. Procédé selon la revendication 3, dans lequel la mise en contact dans l'étape a) et l'incubation dans l'étape b) est conduite en une étape, où la forme intermédiaire du produit alimentaire est incubée dans la solution pendant au moins 5 minutes.
